# EUROPEAN PATENT SPECIFICATION

(11) **EP 3 210 554 B1**
(45) Date of publication and mention of the grant of the patent: **09.02.2022**
(21) Application number: 16157477.7
(22) Date of filing: 25.02.2016
(51) Int. Cl.: A61B 17/70, A61B 17/86

(54) **BONE ANCHOR**
KNOCHENANKER
ANCRAGE OSSEUX

(43) Date of publication of application: 30.08.2017
(60) Divisional application: 21215948.7
(73) Proprietor: Biedermann Technologies GmbH & Co. KG, 78166 Donaueschingen (DE)
(72) Inventor: BIEDERMANN, Timo, 78647 Trossingen (DE); MATTHIS, Wilfried, 79367 Weisweil (DE)
(74) Representative: Prüfer & Partner mbB Patentanwälte · Rechtsanwälte

(56) References cited:
- WO-A1-2006/108329
- WO-A1-2011/054122
- DE-A1-102014 107 882
- US-A1- 2008 039 846
- US-B2- 8 690 930

## Description

The invention relates to a bone anchor having a shank with a channel extending therethrough and a narrowing member provided in the channel at or close to one end of the channel for reducing a cross-section of the channel. The bone anchor can be particularly used in minimally invasive surgery for the treatment of weak and/or osteoporotic bones or vertebrae.

US 8,690,930 describes a bone anchor including a shaft having a first end and a second end, a bore extending from the first end to the second end, and a plug member which is insertable into the bore and guidable through the bore for closing the bore at the second end.

WO 2012/146744 A2 describes a bone screw with a centrally arranged opening and a plurality of openings radial thereto. Bone cement can be injected into the bone through these openings. In order to avoid a cement distribution outside of the bone, a closure apparatus for cementable bone screws is proposed. In one embodiment the closure apparatus is a re-closable sleeve arranged in the bone screw and having the function of a valve. Thereby, the insertion of closure member after having screwed the bone screw into the bone becomes unnecessary.

WO 2011/054122 A1 discloses a medical device comprising a sheath element having longitudinal bore defining a longitudinal opening and a plurality of holes in a wall of the opening. A liquefiable element including a thermoplastic component is inserted into the longitudinal opening and which is liquefiable by the impact of energy (vibration), so that liquefied material flows through the holes into adjacent structures of hard tissue. A distal hole may be provided in a distal end portion of the medical device, in order to assist in pressing out liquefiable material depending on the requirements. By experiments, a cross section and a length of the distal hole can be determined, the ratio of which defines a threshold below which outflow of liquefiable material may be reliably prevented.

The known bone anchors are designed to prevent an escape of bone cement at the tip of the bone anchor by closing the channel at the tip. For certain applications this may be advantageous. For other applications it may be not desirable or even not necessary to close the channel at the tip.

It is the object of the invention to provide a bone anchor that is suitable for use in minimally invasive surgery for the treatment of osteoporotic and/or weak bone.

The object is solved by a bone anchor according to claim 1. Further developments of the bone anchor are given in the dependent claims.

A narrowing member is provided in the channel that extends through the bone anchor at or close to a free end or tip end of the bone anchor which prevents injected bone cement from exiting through the free end. The bone anchor can be used in a minimally invasive surgical procedure wherein the bone anchor is guided to the implantation site by means of a guide wire, such as a Kirschner wire, that is led through the channel. Because the narrowing member reduces a cross-section of the channel without closing the channel, it is possible to pass the guide wire through the bone anchor when the narrowing member has already been placed into the channel or is already present in the channel. As a consequence, a step of closing the channel with a plug after placement of the bone anchor and before injecting the bone cement is not necessary.

The narrowing member in the channel permits to re-introduce the guide wire before injecting the bone cement.

After removal of the guide wire bone cement can be injected which can exit through openings in the wall of the bone anchor but is prevented from exiting through the free end.

The narrowing member may be a separate part that can be easily manufactured and then mounted to the bone anchor. By means of this a modular system can be provided that comprises a variety of shanks having, for example, different channel widths that can be combined with suitable narrowing members with different sizes of the opening for different narrowing effects. A suitable bone anchor can be assembled on demand, dependent inter alia on the viscosity of the bone cement or other substance to be injected.

Alternatively, the narrowing member may be formed in a monolithic manner with the shank of the bone anchor. In this case, the bone anchor comprises less parts but achieves the same functionality.

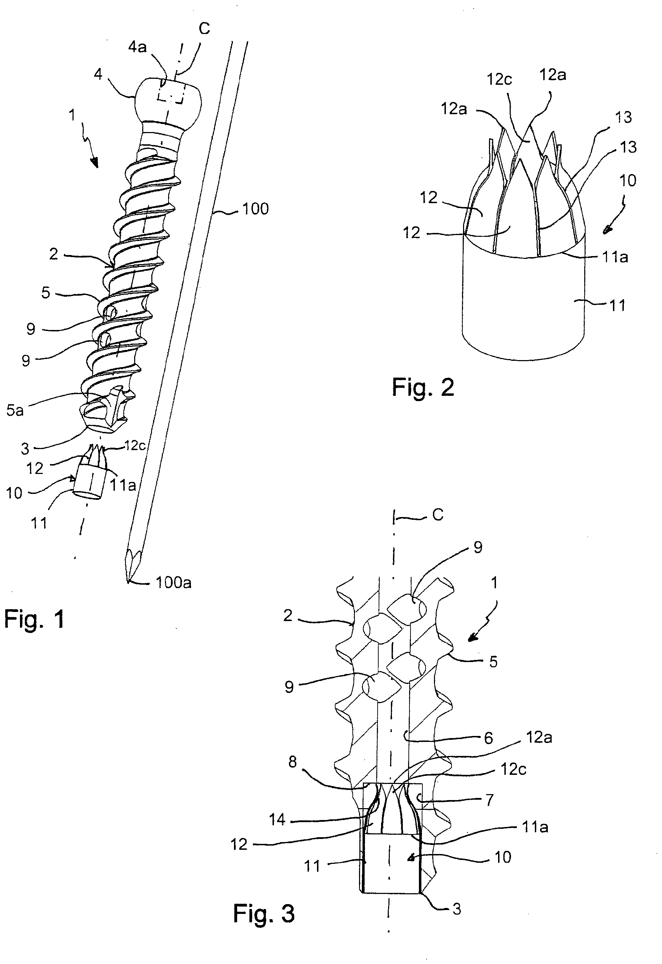

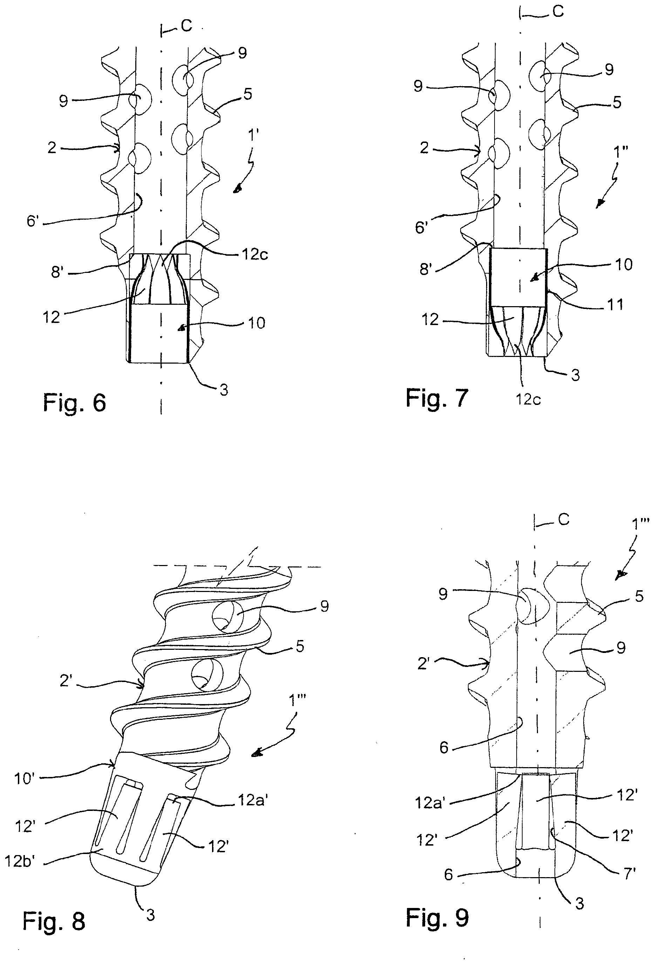

Only the bone anchors shown in Figs. 1-6 are embodiments of the present invention, whereas the bone anchors shown in Figs. 7-12 do not fall within the scope of the appended claims.

Further features and advantages of the invention will become apparent from the description of embodiments by means of the accompanying drawings.

In the drawings:
- Fig. 1:: shows an exploded perspective view of a first embodiment of the bone anchor together with a guide wire.
- Fig. 2: shows a perspective view of the narrowing member of the bone anchor according to the first embodiment shown in Fig. 1.
- Fig. 3: shows a cross-sectional view of a lower portion of the bone anchor including the narrowing member, the cross-section taken in a plane containing a longitudinal axis of the bone anchor.
- Figs. 4a: shows cross-sectional views of the lower portion of the bone anchor according to
- to 4e: Figs. 1 to 3 in several steps using a guide wire.
- Fig. 5: shows a schematic view of a bone anchor according to the first embodiment inserted into the pedicle of a vertebra and filled with bone cement.
- Fig. 6: shows a cross-sectional view of the lower portion of a bone anchor according to a modified first embodiment.
- Fig. 7: shows a cross-sectional view of the lower portion of a bone anchor according to a further modification of the first embodiment.
- Fig. 8: shows a perspective view of the lower portion of a bone anchor according to a second example.
- Fig. 9: shows a cross-sectional view of the lower portion of the bone anchor of Fig. 8, the cross-section taken in a plane containing the longitudinal axis of the bone anchor.
- Fig. 10: shows a perspective view of the lower portion of a bone anchor according to a third example.
- Fig. 11: shows a cross-sectional view of the lower portion of the bone anchor shown in Fig. 10, the cross-section taken in a plane containing the longitudinal axis of the bone anchor.
- Fig. 12: shows a perspective view with a portion shown partially in section of the bone anchor of Figs. 10 and 11.

Figs. 1 to 5 illustrate a first embodiment of a bone anchor. The bone anchor 1 comprises a shank 2 having a free end 3, which may be shaped as a tip, and a head 4 opposite to the free end 3. The head has a shape of a spherical segment and usually has a recess 4a at its free end surface for engagement with a tool. A bone thread 5 is provided on at least a portion of the outer surface of the shank 2. Adjacent to the free end 3, one or more longitudinal cutting notches 5a may be provided in the case of a bone anchor with a self-cutting tip.

The bone anchor 1 is cannulated, i.e. has a channel 6 extending completely through the head 4 and the shank 2 up to the free end 3. More in detail, the channel 6 may be formed by a coaxial bore having a first inner diameter and having a bore axis that forms a longitudinal axis C of the channel 1 and the bone anchor 1. In a portion 7 adjacent to the free end 3, a section with a second inner diameter greater than the first inner diameter of the channel 6 is provided which forms an accommodation space 7 for a narrowing member explained below. Between the accommodation space 7 and the portion of the channel 6 having the first inner diameter a shoulder 8 is formed that acts as a stop for the insertion of the narrowing member.

In the wall of the shank 2 a plurality of openings 9 are provided which connect the channel 6 with the outside. The number, size and arrangement of the openings 9 is designed according to the overall dimensions of the bone anchor 1 for the purpose of forming outlets for bone cement and/or a pharmaceutical substance to be introduced into the bone anchor 1. The openings 9 may be arranged at any position. Preferably, the openings 9 are in a region closer to the free end 3 of the shank 2 than to the head 4. Moreover, the openings 9 may be arranged between the crests of the bone thread 5.

The first inner diameter of the channel has such a size that a guide wire 100, such as a Kirschner wire, which is commonly used for minimally invasive surgery, can be guided through the bone anchor 1. The guide wire 100 may have a tip 100a.

As illustrated more in detail in Figs. 2 and 3, the narrowing member 10 is a sleeve-like piece with a first tubular portion 11 that has a maximum outer diameter sufficient for the sleeve-like piece to be mountable in a press-fit manner into the accommodation space 7 of the bone anchor 1. Adjacent to an edge 11a of the tubular portion 11, a plurality of wall portions 12 having free ends 12a, respectively, are circumferentially arranged with their free ends 12a facing away from the tubular portion 11. Between the wall portions 12 slits 13 are provided. The wall portions 12 have a shape that may be obtained by cutting a hollow sphere-shaped or dome-shaped closed end of the tubular portion 11 at equidistant sections from an outermost end point in the direction of the edge 11a of the tubular portion. Hence, as can be seen in particular in Fig. 3, each flexible wall portion 12 has a base 12b adjacent to the edge 11a of the tubular portion 11 and narrows towards a substantially triangular tip portion 12c adjacent to the free end 12a. The wall portions 12 are bent from their base 12b towards the longitudinal axis C up to a transition portion 14 where the triangular tip portion 12c starts. The triangular tip portions 12c are slightly bent away from the longitudinal axis C. By means of this shape the narrowing member 10 has a reduced opening at the end facing away from the tubular portion.

The wall thickness of the wall portions 12 and the size of the slits 13 is selected such that the wall portions 12 are elastically deformable. Upon the action of a radial pressure from inside the narrowing member 10, the wall portions 12 are configured to move from a resting position outward with respect to the longitudinal axis C thereby increasing the opening at the transition portion 14 and to move back into the resting position when the pressure is released, thereby narrowing the opening again. Similarly, upon the action of a radial pressure from outside onto the wall portions 12, in particular onto the triangular tip portions 12c, the wall portions 12 are configured to move from their resting position towards the longitudinal axis C and move back to the resting position when the pressure is released. For example, the size of the opening defined by the wall portions 12 is such that the wall portions are configured to expand when a guide wire or an instrument is guided through the narrowing member 10 and are configured to snap back to their resting position when the guide wire or the instrument is removed.

When the wall portions 12 are in their resting position, the narrowing member 10 has at least at the transition portion 14 an inner diameter that is smaller than the first inner diameter of the channel 6. Therefore, when the narrowing member 10 is inserted into the accommodation space 7, the channel 6 is narrowed at the transition portion 14 as depicted in Fig. 3. A total axial length of the narrowing member 10 corresponds to an axial length of the accommodation portion 7. The narrowing member 10 can be arranged in the accommodation space in two different orientations. In Fig. 3, the narrowing member is oriented with the triangular tip portions 12c facing towards the head 4 of the bone anchor. The tip portions 12c are slightly bent outward to such an extent that they abut in the inserted condition against the stop provided by the shoulder 8. It is also possible, but not according to the present invention, to orient the narrowing member with the triangular tip portions 12c facing towards the free end 3 of the bone anchor 1.

The bone anchor 1 including the narrowing member 10 may be made from a bio-compatible material, for example of titanium or stainless steel, of a bio-compatible alloy, such as β-titanium or NiTi-alloys, for example Nitinol, of magnesium or magnesium alloys or from a bio-compatible plastic material, such as, for example, polyether ether ketone (PEEK) or poly-1-lactide acid (PLLA). The parts can be made of the same or of different materials. In particular, the narrowing member 10 can be made of a material exhibiting high elasticity, such as Nitinol in the superelastic condition.

In the first embodiment, the narrowing member 10 is mounted to the shank by press-fitting the narrowing member 10 into the accommodation space 7 such that the triangular tip portions 12c are facing towards the head 4 of the bone anchor 1. The assembly of the parts can take place at the manufacturer or can be carried out directly before or during the surgery.

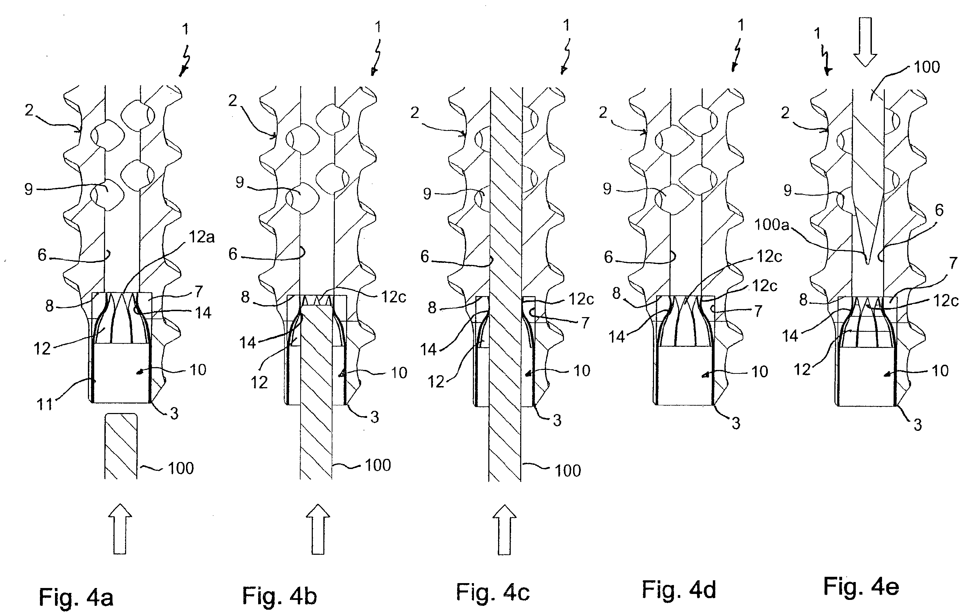

Use of the bone anchor is now described with respect to Figs. 4a to 4e. In a first step, the guide wire 100, such as a Kirschner wire, is placed percutaneously through the skin to the final position of the bone anchor 1 in a bone part or a vertebra of the vertebral column. Thereafter, as shown in Fig. 4a, the bone anchor 1 with the narrowing member 10 is placed with its free end 3 onto the guide wire 100. As depicted in Fig. 4b, the guide wire 100 enters the narrowing member 10 and is guided through the tubular portion 11 until it reaches the transition portion 14 to the triangular tip portions 12c. An outer diameter of the guide wire 100 is greater than the inner diameter of the channel at the transition portion 14 such that the wall portions 12 are slightly pressed outward. As shown in Fig. 4c, the bone anchor 1 is then guided by the guide wire 100 that passes through the channel 6 and screwed into the bone part or the vertebra, for example the pedicle of a vertebra. Thereafter, as illustrated in Fig. 4d, the guide wire 100 is removed. When the flexible wall portions 12 are no longer spread apart by the guide wire, they can return to their resting position in which they narrow the diameter of the channel 6. The guide wire may be re-introduced into the bone anchor as shown in Fig. 4e as long as the bone cement has not yet been injected. Since the triangular end portions 12c of the wall portions 12 are slightly bent outward the tip 100a of the guide wire 100 can easily be inserted into the narrowing member 10.

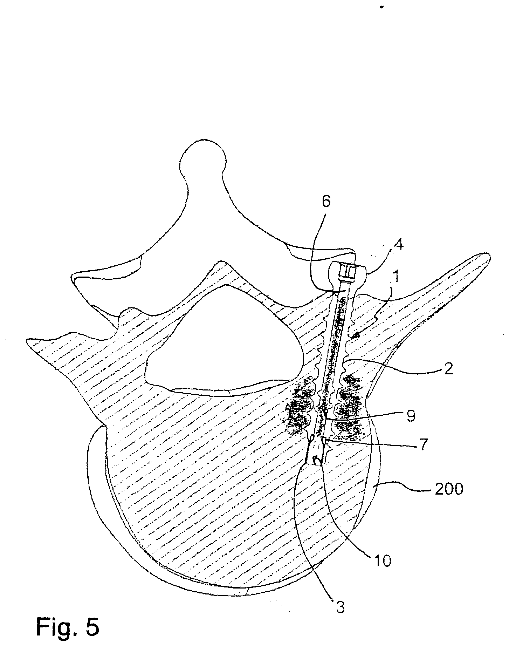

Fig. 5 shows the bone anchor 1 inserted into the pedicle of a vertebra 200. As shown in Fig. 5, bone cement is injected through the head 4 into the channel 6 of the bone anchor 1. The bone cement can exit through the transverse openings 9. When the bone cement encounters the narrowing member 10, the narrowed channel prevents the bone cement from exiting through the free end 3. A complete closure of the channel is not necessary.

In an example of stabilizing the vertebral column, at least two bone anchors are implanted in adjacent vertebrae and connected by a stabilizing member, such as a rod. In this case, the head 4 of the bone anchor can be connected to a receiving part of a polyaxial screw.

A modification of the first embodiment is shown in Fig. 6. The bone anchor 1' comprises a channel 6' that has a greater diameter than the channel of the previous embodiment. The shoulder 8' has a smaller width than that of the previous embodiment. As a result, the triangular tip portions 12c do not abut against the shoulder 8'. The narrowing member 10 is firmly held in the accommodation space 7 by a press-fit connection such that it cannot move further into the channel 6'.

It shall be mentioned that the inner diameter of the accommodation space 7 and of the channel 6' may also be equal.

A further modified example is shown in Fig. 7. The narrowing member 10 is mounted into the accommodation space 7 with the triangular tip portions 12c facing towards the free end 3 of the shank 2. The narrowing member 10 abuts with the tubular portion 11 against the shoulder 8' and is also firmly held by a press-fit connection. The narrowing of the channel 6' at the free end, i.e. in the region of the accommodation space 7, by the narrowing member 10 is sufficient to prevent bone cement from exiting through the free end 3. A guide wire to be inserted can slide along the inner side of the triangular tip portions 12c, since they are slightly bent outward. With the narrowing member 10 being a separate part, it is possible to provide a modular system that includes a plurality of bone anchors 1, 1' with channels 6, 6' having different diameters.

The bone anchor can be selected on demand before or during surgery depending on the medical conditions. It is also possible to provide plurality of narrowing members with different sizes of the wall portions and different sizes of the opening encompassed by the wall portions 12. Hence, depending on several factors, such as the viscosity of the substance to be introduced and the medical condition, a suitable narrowing member 10 can be assembled with a suitable shank 2.

A second example of the bone anchor is shown in Figs. 8 and 9. The narrowing member 10' is formed monolithically with the shank 2'. To accomplish this, the narrowing member 10' forms an end portion of the shank 2'. The channel 6 may extend all through to the free end 3 with the same inner diameter, an end section forming an accommodation space 7' for the narrowing member 10'. In the wall a plurality of circumferentially arranged flexible wall portions 12', made for example by cutting, are provided such that the free ends 12a' thereof are facing towards the head 4 and the bases 12b' thereof are connected to the shank 2'. The flexible wall portions 12' are slightly bent inwards towards the longitudinal axis C, thereby forming the narrowing member that narrows the channel 6. A guide wire (not shown) may be guided from the free end 3 through the channel 6, thereby slightly pressing the flexible wall portions 12' outward. When the guide wire is removed, the flexible wall portions 12' return back to their position narrowing the channel 6. Also in this embodiment, bone cement is prevented from exiting through the free end 3.

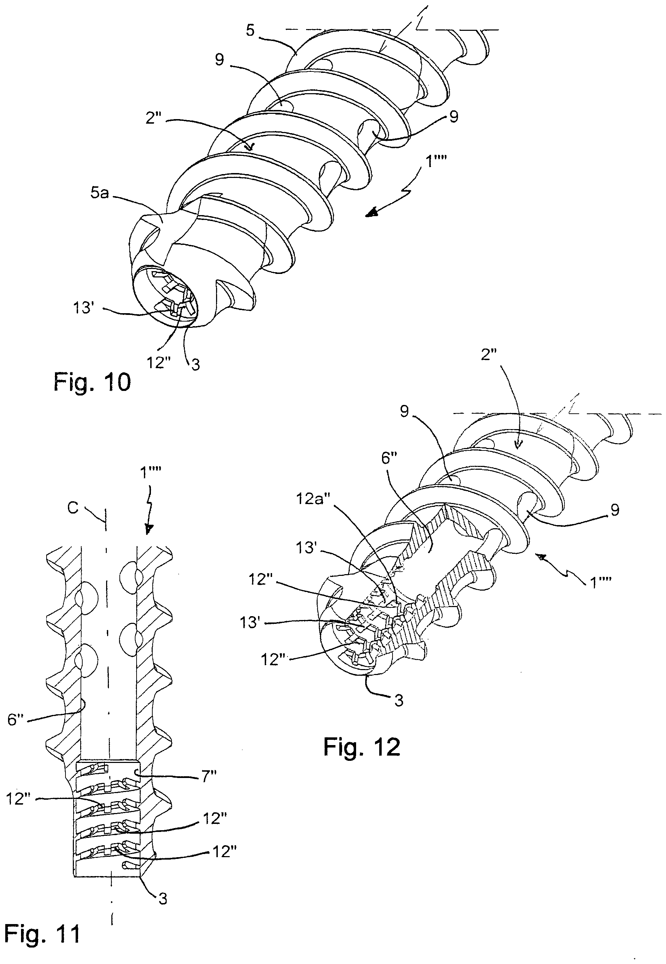

A third example of the bone anchor is shown in Figs. 10 to 12. The narrowing member 1" is also formed in a monolithic manner with the shank 2". The accommodation space 7" has a slightly larger inner diameter than the channel 6 in the main part of the shank. At an inner wall of the accommodation space in a region adjacent to the free end 3, a thread is formed. The flank of the thread facing towards the head 4 forms an undercut, i.e. has a negative flank angle. A plurality of coaxial slots 13' are provided in the crests of the thread such that flexible wall portions 12" remain. By means of the negative flank angle, the free ends 12a" of the flexible wall portions 12" are facing towards the head 4. The flexible wall portions 12" narrow the channel in the region adjacent to the free end 3. Thereby, bone cement is prevented from exiting through the free end 3. The bone anchor 1"" can be preferably made by an additive manufacturing method, such as laser-sintering or laser-melting. This method allows to produce the internal flexible wall portions 12" monolithically with the shank 2" in a simple and fast manner.

It shall be mentioned that the specific shape of the narrowing member 10" is only an example and various other shapes can also be contemplated.

Further modifications of the above-described embodiments are possible. The head 4 can have any other shape or the bone anchor may be a headless bone anchor. The bone anchor can be connected to a receiving part to form a monoaxial or a polyaxial bone screw.

The narrowing member as a separate part can be connected to the shank in other ways than a press-fit connection. It can be connected, for example but not limited to via a threaded connection.

While the bone engagement structure of the embodiments shown is a bone thread, other bone engagement structures can be provided, such as barbs or even the bone engagement structure can be omitted so that the bone anchor is a bone nail.

The number of openings, the size and the shape of the openings 9 may also vary. At least one opening may be sufficient. The channel may have a cross-section varying along the length of the channel.

Other substances, such as pharmaceutical substances may also be injected. The narrowing member with a specific opening is selected in dependency of the viscosity of the substance.

The above embodiments of a bone anchor comprise a shank having a free end, a channel extending through the shank to the free end and having a longitudinal axis C and at least one opening in a wall of the shank which connect the channel with the outside; wherein a narrowing member is provided in the channel in a region at or close to the free end that narrows a cross-section of the channel without closing the channel in such a manner that bone cement or another substance which is introduced into the channel is prevented from exiting through the free end.

## Claims

1. Bone anchor, comprising
a shank (2) having a free end (3),
a channel (6') extending through the shank (2) to the free end (3) and having a longitudinal axis (C) and at least one opening (9) in a wall of the shank which connect the channel with the outside;
wherein a narrowing member (10) is provided in the channel (6) in a region at or close to the free end (3) that narrows a cross-section of the channel without closing the channel;
wherein the narrowing member (10) is at least partially elastically deformable from a first configuration wherein the narrowing member (10) narrows the channel (6) to a first cross section into a second configuration wherein the narrowing member (10) narrows the channel (6) to a second cross-section greater than the first cross section;
wherein the narrowing member (10) comprises at least two wall portions (12), separated from each other, with free ends (12a) that protrude into the channel (6), wherein the free ends (12a) are facing away from the free end (3) of the shank (2),
**characterized in that** the free ends (12a) are bent away from the longitudinal axis (C) of the channel (6).

2. The bone anchor of claim 1, wherein the narrowing member (10) is a separate part that is insertable into the channel (6).

3. The bone anchor of claim 1, wherein the narrowing member (10) is a sleeve-like piece that is preferably press-fit into the channel.

4. The bone anchor of claim 2 or 3, wherein a section of the channel adjacent to the second end (3) has a first cross-section greater than a second cross-section of the channel adjacent to the section thereby forming an accommodation space (7) and wherein the narrowing member (10) is arranged in the accommodation space (7).

5. The bone anchor of claim 4, wherein a space is provided between an outer wall portion of the narrowing member (10) and an inner wall of the accommodation space (7).

6. The bone anchor of one of claims 1 to 5, wherein the narrowing member (10) is configured to permit guiding a guide wire (100) or an instrument to pass therethrough.

7. The bone anchor of claim 6, wherein the narrowing member (10) is configured to be spread when the guide wire (100) or an instrument extends therethrough and to return to a non spread configuration when the guide wire (100) or the instrument is removed.

## Patentansprüche

1. Knochenanker, umfassend:
einen Schaft (2) mit einem freien Ende (3),
einen Kanal (6'), der sich durch den Schaft (2) zu dem freien Ende (3) erstreckt und eine Längsachse (C) sowie wenigstens eine Öffnung (9) in einer Wand des Schafts besitzt, die den Kanal mit einer Außenumgebung verbindet;
wobei in dem Kanal (6) ein Verengungsteil (10) in einem Bereich an oder nahe dem freien Ende (3) vorgesehen ist, der einen Querschnitt des Kanals verengt ohne aber den Kanal zu verschließen;
wobei der Verengungsteil (10) zumindest teilweise aus einer ersten Konfiguration, in welcher der Verengungsteil (10) den Kanal (6) zu einem ersten Querschnitt verengt, in eine zweite Konfiguration elastisch verformbar ist, in welcher der Verengungsteil den Kanal (6) zu einem zweiten Querschnitt verengt, der größer ist als der erste Querschnitt;
wobei der Verengungsteil (10) zumindest zwei Wandabschnitte (12) umfasst, die getrennt voneinander sind, mit freien Enden (12a), die in den Kanal (6) hervorstehen, wobei die freien Enden (12a) von dem freien Ende (3) des Schafts (2) weggerichtet sind,
**dadurch gekennzeichnet, dass** die freien Enden (12a) von der Längsachse (6) des Kanals (6) weg gebogen sind.

2. Der Knochenanker gemäß Anspruch 1, wobei der Verengungsteil (10) ein separates Teil ist, das in den Kanal (6) einführbar ist.

3. Der Knochenanker gemäß Anspruch 1, wobei der Verengungsteil (10) ein hülsenähnliches Teil ist, das vorzugsweise in den Kanal pressgepasst ist.

4. Der Knochenanker gemäß Anspruch 2 oder 3, wobei ein zu dem zweiten Ende (3) angrenzender Abschnitt des Kanals einen ersten Querschnitt besitzt, der größer ist als ein zweiter Querschnitt des Kanals angrenzend an den Abschnitt, wobei der Abschnitt einen Aufnahmeraum (7) ausbildet und wobei das Verengungsteil (10) in dem Aufnahmeraum (7) angeordnet ist.

5. Der Knochenanker gemäß Anspruch 4, wobei ein Raum zwischen einem Außenwandabschnitt des Verengungsteils (10) und einer Innenwand des Aufnahmeraums (7) vorgesehen ist.

6. Der Knochenanker gemäß einem der Ansprüche 1 bis 5, wobei der Verengungsteil (10) eingerichtet ist, eine Führung eines Führungsdrahts (100) oder ein Hindurchtreten eines Instruments da hindurch zu erlauben.

7. Der Knochenanker gemäß Anspruch 6, wobei der Verengungsteil (10) eingerichtet ist, gespreizt zu werden, wenn sich der Führungsdraht (100) oder ein Instrument da hindurch erstreckt, und in eine nicht-gespreizte Konfiguration zurückzukehren, wenn der Führungsdraht (100) oder das Instrument entfernt werden.

## Revendications

1. Un ancrage osseux, comprenant
une tige (2) ayant une extrémité libre (3),
un canal (6') s'étendant à travers la tige (2) jusqu'à l'extrémité libre (3) et ayant un axe longitudinal (C) et au moins une ouverture (9) dans une paroi de la tige qui relie le canal avec l'extérieur ;
dans lequel un élément de rétrécissement (10) est prévu dans le canal (6) dans une région au niveau ou à proximité de l'extrémité libre (3) qui rétrécit une section transversale du canal sans fermer le canal ;
dans lequel l'élément de rétrécissement (10) est au moins partiellement déformable élastiquement à partir d'une première configuration dans laquelle l'élément de rétrécissement (10) rétrécit le canal (6) à une première section transversale, à une seconde configuration dans laquelle l'élément de rétrécissement (10) rétrécit le canal (6) à une seconde section transversale plus grande que la première section transversale;
dans lequel l'élément de rétrécissement (10) comprend au moins deux parties de paroi (12), séparées l'une de l'autre, avec des extrémités libres (12a) qui font saillie dans le canal (6), dans lequel les extrémités libres (12a) sont orientées dans une direction s'éloignant de l'extrémité libre (3) de la tige (2),
**caractérisé en ce que** les extrémités libres (12a) sont pliées dans une direction s'éloignant de l'axe longitudinal (C) du canal (6).

2. L'ancrage osseux selon la revendication 1, dans lequel l'élément de rétrécissement (10) est une pièce séparée qui est insérable dans le canal (6).

3. L'ancrage osseux selon la revendication 1, dans lequel l'élément de rétrécissement (10) est une pièce en forme de manchon qui est de préférence insérée par ajustage serré dans le canal.

4. L'ancrage osseux selon la revendication 2 ou 3, dans lequel une section du canal adjacente à la seconde extrémité (3) a une première section transversale plus grande qu'une seconde section transversale du canal adjacente à la section, formant ainsi un espace de logement (7), et dans lequel l'élément de rétrécissement (10) est disposé dans l'espace de logement (7).

5. L'ancrage osseux selon la revendication 4, dans lequel un espace est prévu entre une partie de paroi extérieure de l'élément de rétrécissement (10) et une paroi intérieure de l'espace de réception (7).

6. L'ancrage osseux selon l'une des revendications 1 à 5, dans lequel l'élément de rétrécissement (10) est configuré pour permettre le guidage d'un fil de guidage (100) ou d'un instrument pour passer à travers celui-ci.

7. L'ancrage osseux selon la revendication 6, dans lequel l'élément de rétrécissement (10) est configuré pour s'étaler lorsque le fil de guidage (100) ou un instrument s'étend à travers celui-ci et pour revenir à une configuration non étalée lorsque le fil de guidage (100) ou l'instrument est retiré.
